# EUROPEAN PATENT APPLICATION

(11) **EP 2 298 428 A1**
(43) Date of publication of application: **23.03.2011**
(21) Application number: 10012583.0
(22) Date of filing: 21.12.2000
(51) Int. Cl.: B01D 29/00, B01D 35/00, B01D 35/01, A61M 1/36

(54) **Fluid filtration system**

(62) Divisional of application: 00988255.6
(71) Applicant: Hemerus Medical, LLC, St. Paul, MN 55112 (US)
(72) Inventor: Zia, Majid, White Bear Township Minnesota 55127 (US); Khamis, Chaouki A., Minneapolis Minnesota 55432 (US)
(74) Representative: Pfenning, Meinig & Partner GbR

(57) **Abstract**

A biological fluid filter is shown for filtering biological products into their therapeutically valuable components. The biological fluid filter (20) can use a wide variety of filter media and filter a wide variety of biological fluids without being limited by the CWST of the media. The construction comprises an inlet section (21) and an outlet section (22) with a filter media (39) separating the inlet section (21) from the outlet section (22). The construction may also have a first vent chamber (27) and a second vent chamber (30).

## Description

The present invention relates to a biological fluid filter for use in filtering biological fluid products into their therapeutically valuable components. The filtration media used in the biological fluid filters of the prior art are chosen or modified in order to have a critical wetting surface tension (CWST) in the proximity of the surface tension (ST) of the biological fluid to be filtered. If the CWST of the media is substantially lower that the ST of the biological fluid being filtered, the priming time for the filter increases as the difference between the CWST and the ST increases.

If the CWST of the media is substantially higher that the ST of the fluid, the filter media is easily wetted by the biological fluid. In cases where the filter media is easily wetted by the biological fluid, air may be entrapped within the filter device, which results is a reduction of the usable filter area, and decreased performance.

In the present invention, a filter device is introduced which alleviates or minimizes gas entrapment. The device is not limited by the CWST of the filter media, or the ST of the fluid to be filtered. Also, the device improves the filter priming time.

These benefits are achieved by dividing the filter medium into one or more sections, separated from each other, so that the wicking properties of the filter medium used will not cause any substantial air entrapment in the biological fluid filter device.

### BRIEF DESCRIPTION OF THE DRAWINGS

Fig. 1 is a perspective view of a biological fluid filter construction embodying the present invention;
Fig. 2 is a front elevational view of the construction shown in Fig. 1;
Fig. 3 is a sectional view, taken in the direction of the arrows, along the section line 3-3 of Fig. 2;
Fig. 4 is a sectional view, taken in the direction of the arrows, along the section line 4-4 of Fig. 2;
Fig. 5 is a sectional view, taken in the direction of the arrows, along the section line 5-5 of Fig. 2;
Fig. 6 is a front elevational view of the inlet portion of the construction shown in Fig. 1;
Fig. 7 is a rear elevational view of the construction shown in Fig. 6;
Fig. 8 is a front elevational view of the outlet portion of the construction shown in Fig. 1;
Fig. 9 is a rear elevational view of the construction shown in Fig. 8;
Fig. 10 is a modification of the construction shown in Fig. 1;
Fig. 11 is a diagrammatic view of the filter medium shown in the construction of Fig. 10;
Fig. 12 is a diagrammatic view of a further modification of the construction shown in Fig. 1; and
Fig. 13 is a diagrammatic view of the filter medium shown in the construction shown in Fig. 12.

It is to be understood that the present invention is not limited in its application to the details of construction and arrangement of parts illustrated in the accompanying drawings, since the invention is capable of other embodiments, and of being practiced or carried out in various ways within the scope of the claims. Also, it is to be understood that the phraseology and terminology employed herein is for the purpose of description, and not of limitation.

### DESCRIPTION OF THE PREFERRED EMBODIMENT

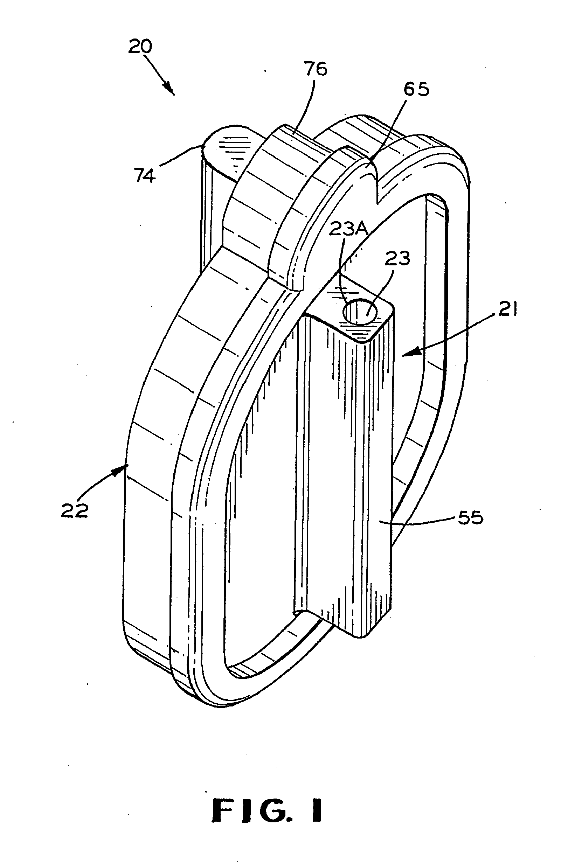

Referring to Fig. 1, there is shown a biological fluid filter, generally indicated by the numeral 20. The biological fluid filter device 20 may be such as is used in a system for processing a biological fluid, such as blood, into its therapeutically valuable components. The present invention is particularly useful for preventing gas entrapment in the upstream or inlet chamber of a biological fluid filter.

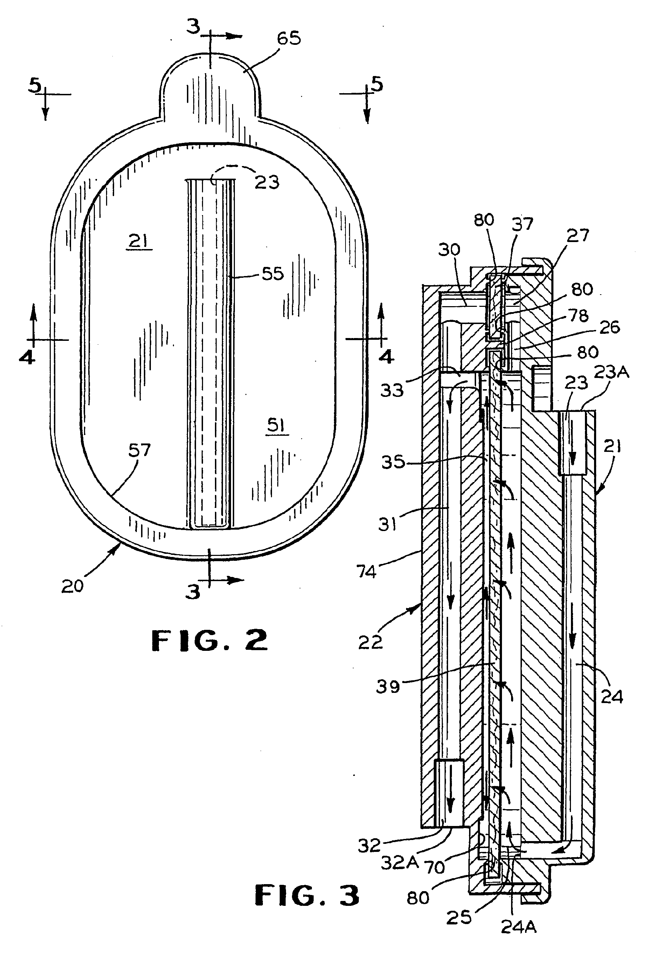

The biological fluid filter 20 consists of an inlet section 21 and an outlet section 22. Referring to Figs. 2 and 3, the inlet section 21 of biological fluid filter 20 has an inlet 23, including port 23A, communicating with first passage 24, which is in fluid communication with first or inlet chamber 25 through first port or outlet 24A. Further, biological fluid filter 20 has a second passage 26 in fluid communication with both, first or inlet chamber 25, and first vent chamber 27.

The outlet section 22 has a second vent chamber 30 in fluid communication with a third passage 31. Third passage 31 is in fluid communication with outlet 32 through port 32A. A fourth passage 33 is in communication with the third passage 31 and the second or outlet chamber 35. A vent filter element 37 separates the first vent camber 27 from the second vent chamber 30, and is held in place by means to be described in more detail hereinbelow.

Similarly, a biological filter element 39 separates the first or inlet chamber 25 from the second or outlet chamber 35. Both the vent filter element 37 and the biological filter element 39 may consist of one or more layers, and be made of a wide variety of filter materials. In the embodiment illustrated, they are liquiphilic.

In the preferred embodiment, the vent filter element 37, and the biological fluid filter element 39, are made of the same filter medium, which may be such as glass or nylon fibers. In use, a fluid container (not shown), such as a blood container is placed in fluid communication with inlet port 23A. Similarly, a biological fluid receiving bag (not shown) is placed in fluid communication, by means well known in the art, with outlet port 32A. Fluid flow is initiated and blood flows in the inlet port 23A, through the first passage 24 and through first outlet 24A into inlet chamber 25.

In operation, as the blood enters the inlet chamber 25, the blood may wick into the filter element 39. The blood may wick into the filter element 39 faster, or slower, than the blood level rises in the first or inlet chamber 25. The rate at which the blood wicks into the filter element 39 will depend on the properties of the filter medium being chosen, and the biological fluid being filtered. These properties include the pore size of the medium, the density of the biological fluid, the surface tension of the biological fluid, and the contact angle of the solid-liquid-gas interface.

While the blood level is rising in the inlet chamber 25, any air entrapped in chamber 25 is either passing through a portion of the filter element 39 which is not yet wetted, or is proceeding through second passage 26 and being vented out the vent filter element 37.

As the blood level continues to rise in inlet chamber 25, at some point, the biological filter element 39 will be sufficiently "wetted", and the biological fluid being filtered will "breakthrough" the filter element 39, and will start flowing into outlet chamber 35. The fluid breakthrough depends on the pore size of the material; the surface tension and the contact angle, as well as the pressure differential across the filter element 39.

Due to the pressure differential across the biological filter element 39, the biological fluid continues to flow up into second passage 26. If the pressure differential is sufficient, the biological fluid will contact the vent filter element 37, which is the preferred embodiment. If the vent filter element 37 is also made of a liquiphilic media, it will become "wetted out". However, by this time all the gas entrapped in inlet chamber 25 has either passed previously through biological filter element 39 or through vent filter element 37 and accomplished one of the objects of the invention.

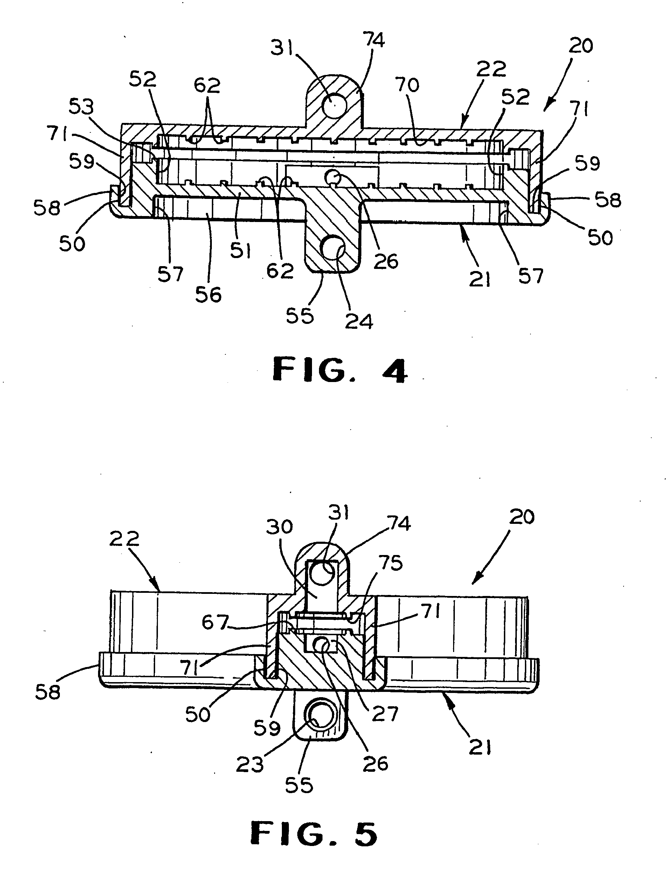

Referring now to Figs. 2-7, the construction of the inlet section 21 of the biological fluid filter 20 may be clearly understood. The biological fluid filter 20 includes an inlet section 21 which is bonded to an outlet section 22 by a seal 50. The seal 50 is preferably an ultrasonic seal. It can be understood by those skilled in the art that other seals such as heat seals, adhesive seals, or any other air tight seal may be used.

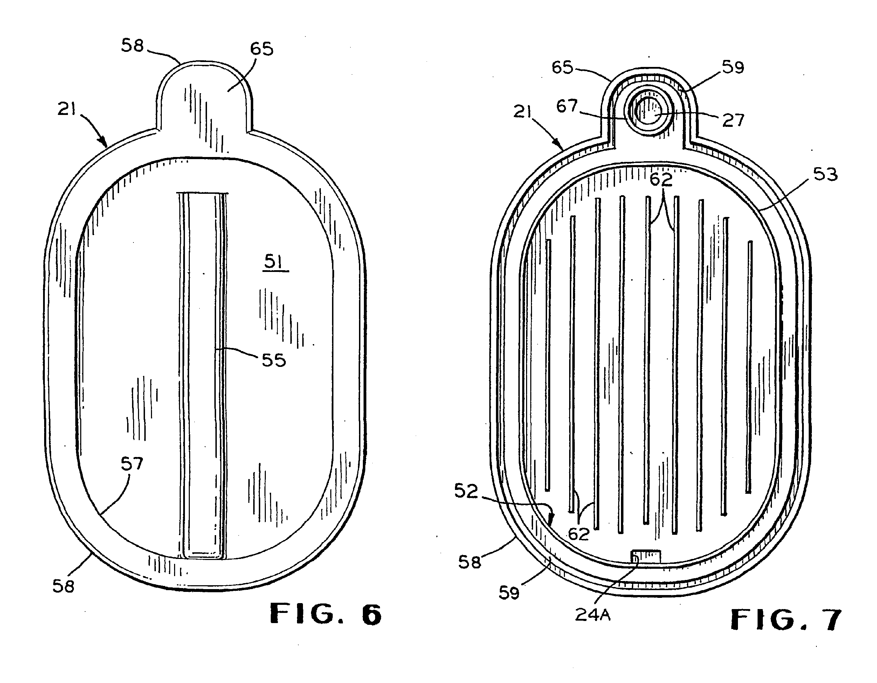

Inlet section 21 includes a recessed top wall 51, and downstanding side walls 52 extending around the periphery of the recessed top wall 51. A downstanding peripheral ridge 53 extends around the periphery of the downstanding side wall 52 and forms a part of the mechanism which holds the vent filter element 37 and the biological filter element 39 in place, as will be more fully explained hereinafter. A first protuberance 55 extends from the recessed top wall 51, and carries the inlet 23 and first passage 24 as previously described. First or outlet port 24A which is in fluid communication with the first passage 24 can be seen in Fig. 7. A recess 56, provided by the combination of the top surface of the recessed top wall 51 and the peripheral side walls 57 almost completely surrounds the protuberance 55.

A peripheral flange 58 depends from the peripheral sidewall 57 and forms a groove 59 extending around the periphery of the inlet section 21 of the biological fluid filter 20. The groove 59 forms a portion of the means by which the seal 50 between the inlet section 21 and the outlet section 22 of the biological fluid filter 20 is formed. A plurality of downstanding ribs 62 are provided on the lower surface of the recessed top wall 51 for purposes to be described.

The inlet portion 21 of the biological fluid filtration device also has an extended portion 65 which contains second passage 26 (Fig. 3) in fluid communication with first vent chamber 27. The same flange 58 and groove 59 are provided in the extended portion 65 of the inlet section 21 as are provided in the remainder of the inlet section 21, so that the inlet section 21 will properly mate with the outlet section 22 to be described. A circular ridge 67 is provided about the first vent chamber 27 to aid in holding the vent filter, as will be further described.

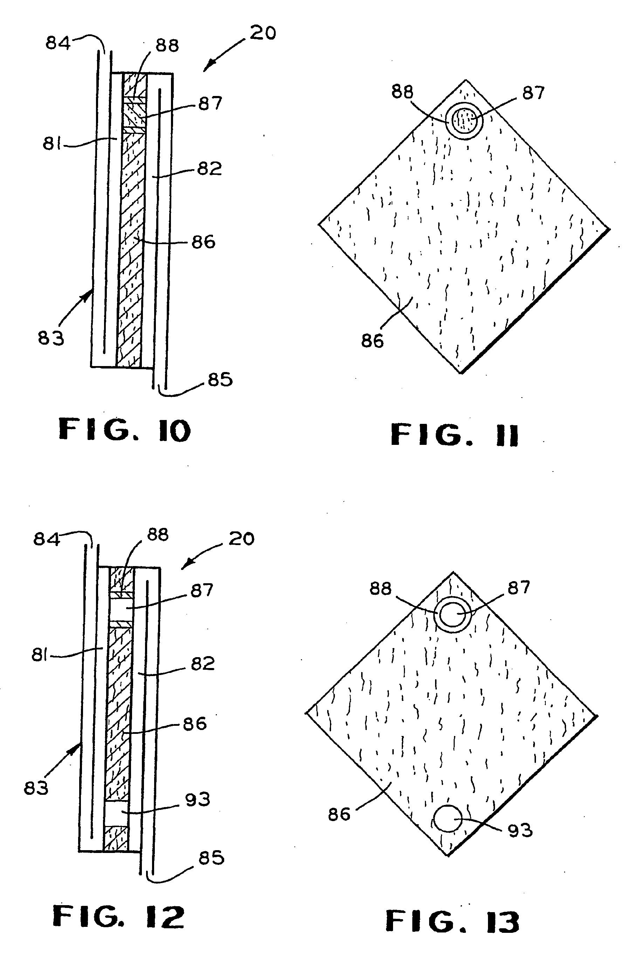

Referring now to Figs. 3-5 and 8-9, the construction of the outlet portion 22 of the biological fluid filter 20 will be clearly understood. The shape of the outlet section 22 of the biological fluid filter 20 is complimentary in shape to the inlet section 21 so that the inlet section 21 may act as a closure to the outlet section 22, or vice versa. It can easily be understood by those skilled in the art that the biological fluid filter 20 may be of any desired shape, such as the generally oval shape thus far described, the diamond shape of the modification shown in Figs. 10 or 12, or any other desired shape. Similar to the inlet section 21, the outlet section 22 of the biological fluid filter 20 has a bottom wall 70 and upstanding sidewall 71. The top of the upstanding sidewall 71 fits into the groove 59 in the inlet portion 21, and is preferably sonically welded to form the seal 50. A second protuberance 74 is provided on the exterior portion of the bottom wall 70 and carries the third passage 31, fourth passage 33, and a portion of the vent chamber 30. A second circular ridge 75, complimentary in shape to the circular ridge 67, is provided. The protuberance 74 covers a portion of the extended portion 76 of the outlet portion 22 of the biological fluid filter 20.

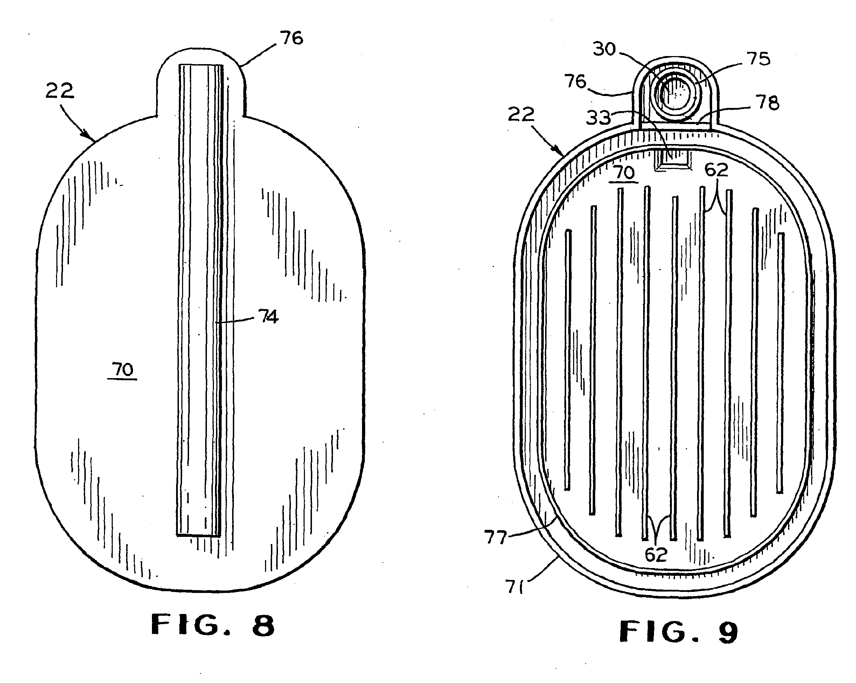

As seen in Fig. 9, a further plurality of ribs 62 is provided on the interior surface of the bottom wall 70 to help support the biological filter element 39 and provide flow in the second or outlet chamber 35 of the biological fluid filter 20. An upstanding ridge 77 is provided in a spaced apart relationship to the upstanding sidewall 71. As with the circular ridges 67 and 75, when the outlet portion 22 and the inlet portion 21 are in mating relationship, the downstanding ridge 53 and the upstanding ridge 77 will be in a 180° opposed relationship. As can be seen in Fig. 3 these ridges will provide the pinch seals 80 for the vent filter element 37 and the biological filter element 39. An ultrasonic weld ridge 78 is provided to separate vent filter 37 and biological filter element 39, and to provide additional support for the fluid filter 20.

Referring to Figs. 10 and 11, there is shown a modification of the biological fluid filter 20 previously described. In this modification of the invention, the biological fluid filter 20 has a housing 83, which may be constructed in a manner similar to that just described, or may be constructed by other means well known in the art. The housing has an inlet 84 to which a biological fluid container of the type well known in the art would be in fluid communication during operation. The housing 83 also has an outlet 85 through which the filtered fluid passes. The outlet 85 would be in fluid communication with a biological receiving container (not shown) .

A filter element 86 would be sealingly mounted within the housing between inlet 84 and outlet 85. In this modification of the biological fluid filter 20, instead of there being a separate and distinct vent filter element 37, the vent filter element 37 is embedded in the biological fluid filter element 86. The biological filter element 86 may be made of a liquiphilic filter medium, and the embedded vent filter element 87 may also be made of a liquiphilic filter medium, surrounded by a solid or liquiphobic barrier 88. In operation, this modification of the biological fluid filter would operate in a similar manner to that just described because of the liquiphilic nature of the biological filter element 86, until the element was completely saturated. As the blood was rising in the inlet chamber 81, any entrapped gas would pass through the embedded vent filter element 87 until the level of the blood surpassed the solid or liquiphobic barrier 88. At this time, virtually all of the entrapped gas would be downstream of the biological filter element 86, the element would be completely saturated, and blood would now freely flow into the outlet chamber 82.

Another modification of the biological fluid filter 20 is shown in Figs. 12 and 13. As before, there is a filter housing 83 having an inlet 84 communicating with an inlet chamber 81, and an outlet 85 communicating with an outlet chamber 82. In this modification of the invention, the biological filter element 86 has a first embedded liquiphilic gas vent 87 surrounded by a solid barrier 88, and a second embedded liquiphobic gas vent 93.

In operation, a biological fluid container known in the art (not shown) will be in fluid communication with inlet 84. As blood is released from the biological fluid container it will flow into the inlet chamber 81 and come into contact with the bottom of the biological fluid filter element 86. Since filter element 86 may be a liquiphilic porous medium, the blood level may wick up in the liquiphilic porous medium 86 faster than the level in the chamber 81. The blood will not pass through the liquiphobic second embedded gas vent 93.

The second embedded gas vent 93 will have no effect on the operation of the biological fluid filter 20 while the liquid level continues to rise in inlet chamber 81. However, the difference between the embodiment of the invention shown in Figs. 10 and 11, and 12 and 13, becomes apparent when all of the blood has been released from the biological filter container and the level starts dropping in the inlet chamber 81. The vent filter element 87 will stay wetted out as the level in the inlet chamber 81 drops because of the blood present in the outlet chamber 82. However, as the level in the inlet chamber 81 continues to drop it will drop below the level of the liquiphobic second embedded gas vent 93. Since gas vent 93 did not wet out, when the blood level drops, air will pass from the inlet chamber 81 through the second embedded gas vent 93, and aid in draining the filter element 86, as well as the outlet chamber 82, through the outlet 85.

Thus, a new and novel biological fluid filtration device has been developed which can use a wide range of filter media, and filter a wide range of biological fluids, without being limited by the CWST of the media, or the ST of the biological fluid being filtered.

In the following, further advantageous aspects of the invention are described.

The first aspect relates to a biological fluid filter comprising:
a) an inlet section, said inlet section comprising a top wall and a downstanding peripheral side wall,
b) an outlet section complimentary in shape to and bonded to said inlet section to form a chamber communicating with atmosphere, said outlet section comprising a bottom wall and upstanding sidewall, and
c) a filter element mechanically held in place between said inlet section and said outlet section to divide said chamber into at least an inlet chamber and an outlet chamber.

In the filter defined in the previous aspect, said inlet section can further comprise:
a) an inlet,
b) a first passage in fluid communication with said inlet, and
c) a first outlet in fluid communication with said first passage and said inlet chamber.

In the filter defined in one of the previous aspects, said outlet section can further comprise:
a) a fourth passage in fluid communication with said outlet chamber,
b) a third passage in fluid communication with said fourth passage, and
c) an outlet in fluid communication with said third passage.

Another aspect relates to a biological fluid filter, comprising:
a) an inlet section having an inlet, a first passage in fluid communication with said inlet, a first port in fluid communication with said first passage, an inlet chamber in fluid communication with said first port, and a second passage in fluid communication with said inlet chamber, and a first vent chamber; and
b) an outlet section comprising an outlet chamber,
   a fourth passage in fluid communication with said inlet chamber, a third passage in fluid communication with said fourth passage, and an outlet in fluid communication with said fourth passage;
c) a second vent chamber in fluid communication with said third passage;
d) a first vent chamber in fluid communication with said inlet chamber through a second passage;
e) a vent filter element separating said first vent chamber from said second vent chamber; and
f) a biological filter element separating said inlet chamber from said outlet chamber.

In this biological fluid filter, said inlet section can have an inlet and an inlet port for communication with said first passage.

In this biological fluid filter, said third passage can be in fluid communication with an outlet through an outlet port.

In this biological fluid filter, said vent filter element and said biological filter element can consist of at least one layer.

In this biological fluid filter, said vent filter element and said biological filter element can be liquiphilic.

Said vent filter element and said biological filter element can be made of the same filter medium.

Said inlet section can be bonded to said outlet section by an air tight seal.

Said seal can be an ultrasonic seal.

Said inlet section can include a recessed top wall and a downstanding sidewall peripherally extending from said top wall.

A downstanding peripheral ridge can extend around the periphery of said downstanding sidewall.

In a further aspect, a first protuberance can extend from said recessed top wall, carrying said inlet and said first passage.

A peripheral flange can depend from said peripheral sidewall and can form a groove extending around the periphery of said inlet section.

Said groove can form a portion of the means by which said seal between said inlet section and said outlet section is formed.

Said recessed top wall can be provided with a lower surface.

Said lower surface can be provided with a plurality of downstanding ribs.

Said inlet portion can have an extended portion containing a second passage in fluid communication with said first vent chamber.

Said extended portion can be provided with said flange and said groove for proper mating with said outlet section.

Said first vent chamber can be provided with a circular ridge to aid in holding said vent filter.

Said outlet section can be complimentary in shape to said inlet section allowing said inlet section to act as a closure to said outlet section, or vice versa.

Said outlet section can have a bottom wall and a upstanding sidewall.

Said upstanding sidewall can fit into said groove in said inlet section.

Said upstanding sidewall can be sonically welded to form a seal with said inlet section.

An upstanding ridge can be provided in a space apart relationship to said upstanding sidewall.

When said outlet portion and said inlet portion are in a mating relationship, said upstanding ridge and said downstanding ridge can form pinch seals for said vent filter element and said biological filter element.

Said bottom wall can have an exterior and an interior.

Said exterior of said bottom wall can be provided with a second protuberance.

Said second protuberance can carry said third passage, said fourth passage, and a portion of said second vent chamber.

Said second protuberance can cover a portion of an extended portion of said outlet portion.

A plurality of ribs can be provided on said interior surface of said bottom wall to help support said biological filter element and provide flow in said second chamber.

An ultrasonic weld ridge can be provided to separate said vent filter and said biological filter element and to provide additional support for said filter element.

A further aspect of the invention relates to a biological fluid filter, comprising:
a) an inlet for communication with a biological fluid container;
b) an outlet for communication with a biological
   receiving container for filtered biological fluid;
c) a filter housing containing said inlet and said outlet;
d) a biological filter element sealingly mounted between said inlet and said outlet; and,
e) a vent filter element embedded in said biological filter element.

Said biological filter element can be made of a liquiphilic filter medium.

Said embedded vent filter element can be made of a liquiphilic filter medium surrounded by a solid or liquiphobic barrier.

A further aspect of the invention relates to a biological fluid filter, comprising:
a) an inlet communicating with an inlet chamber;
   an outlet communicating with an outlet chamber;
b) a filter housing containing said inlet and said outlet and said inlet chamber and said outlet chamber;
c) a biological filter element mounted between said inlet and said outlet;
d) a first liquiphilic gas vent embedded in said biological filter element and surrounded by a solid barrier; and,
e) a second liquiphobic gas vent embedded in said biological filter element.

A further aspect of the invention relates to a fluid filtration system including:
a) a filtration device, said filtration device having a filter media dividing said filtration device into an up-stream side and a downstream side,
b) a storage container for holding filtered fluid,
c) a second conduit connected between the downstream side of said filtration device and said storage container, and
d) a second vent line in fluid communication with the downstream side of said filtration device and selectively open to atmosphere.

This fluid filtration system can further include a downstream gas inlet in fluid communication with said second vent line.

In this fluid filtration system, said downstream gas inlet can include;
a) an inlet open to atmosphere,
b) an outlet connected to said second vent line, and
c) at least one layer of a porous medium interposed between said inlet and said outlet.

Said downstream gas inlet can further include a cap for the selective opening and closing of said inlet.

Said at least one layer of a porous medium can be a bacterial retention medium.

Said at least one layer of a porous medium can be a viral retention medium.

A further aspect of the invention relates to a fluid filtration system including:
a) a fluid container,
b) a first conduit in fluid communication with said fluid container,
c) a vent line in fluid communication with said first conduit and having an outlet at a sufficient height so that a fluid being filtered does not exit, and having a portion which prevents gas entry into said fluid filtration system until a desired amount of fluid has exited said fluid container.

This fluid filtration system can further include an upstream gas inlet housing in fluid communication with said vent line.

Said gas inlet housing can include:
a) an inlet open to atmosphere,
b) an outlet connected to said vent line, and
c) at least one layer of a porous medium interposed between said inlet and said outlet.

Said at least one layer of a porous medium can be a bacterial retention medium.

The fluid filtration system defined in claim 46, wherein said at least one layer of a porous medium is a viral retention medium.

A further aspect of the invention relates to a fluid filtration system including:
a) a filtration device, said filtration device having a filter media dividing said filtration device into an up-stream side and a downstream side,
b) a storage container for holding filtered fluid,
c) a second conduit connected between the downstream side of said filtration device and said storage container, and
d) a second vent line in fluid communication with the downstream side of said filtration device and selectively open to atmosphere.

This fluid filtration system can further include:
a) a fluid container,
b) a first conduit in fluid communication with said fluid container,
c) a vent line in fluid communication with said first conduit and having an outlet at a sufficient height so that a fluid being filtered does not exit, and having a portion which prevents gas entry into said fluid filtration system until a desired amount of fluid has exited said fluid container.

A further aspect of the invention relates to a biological fluid filter comprising:
(a) an inlet section,
(b) an outlet section complimentary in shape to, and sealingly connected to said inlet section, and
(c) a filter element held in place between said inlet section and said outlet section to form at least an inlet chamber and an outlet chamber, said filter element comprising a filter medium divided into two or more sections, separated from each other, so that the wicking properties of the filter medium used will not cause any substantial air entrapment in said biological fluid filter.

A further aspect of the invention relates to a fluid filter comprising:
(a) an inlet section,
(b) an outlet section complimentary in shape to, and sealingly connected to said inlet section, and
(c) a filter element held in place between said inlet section and said outlet section to form at least an inlet chamber and an outlet chamber, said filter element comprising a filter medium divided into two or more sections, separated from each other, so that the wicking properties of the filter medium used will not cause any substantial air entrapment in said fluid filter.

A further aspect of the invention relates to a filter element comprised of a filter medium said filter medium divided into two or more sections, separated from each other, so that the wicking properties of the filter medium used will not cause any substantial air entrapment when used in a biological fluid filter device.

A further aspect of the invention relates to a biological fluid filter having an inlet section and an outlet section, a first vent chamber in said inlet section, a second vent chamber in said outlet section, a fourth passage in fluid communication with said outlet section, and a vent filter element separating said first vent chamber from said second vent chamber.

A further aspect of the invention relates to a fluid filter having an inlet section and an outlet section, a first vent chamber in said inlet section, a second vent chamber in said outlet section, a fourth passage in fluid communication with said outlet section, and a vent filter element separating said first vent chamber from said second vent chamber.

A further aspect of the invention relates to a fluid filter comprising:
a) a filter housing, said filter housing having at least one inlet and at least one outlet, and
b) a filter medium separating said filter housing into at least one inlet chamber and at least one outlet chamber, said filter medium comprising at least two sections separated from each other such that substantially no air entrapment occurs within the filter housing.

## Claims

1. A fluid filtration system including:
a) a fluid container,
b) a first conduit in fluid communication with said fluid container,
c) a vent line in fluid communication with said first conduit and having an outlet at a sufficient height so that a fluid being filtered does not exit.

2. The fluid filtration system of claim 1, wherein the vent line has a portion which prevents gas entry into said fluid filtration system until a desired amount of fluid has exited said fluid container.

3. The fluid filtration system defined in claim 1, and further including:
a) an upstream gas inlet housing in fluid communication with said vent line.

4. The fluid filtration system defined in claim 3, wherein said gas inlet housing includes:
a) an inlet open to atmosphere,
b) an outlet connected to said vent line, and
c) at least one layer of a porous medium interposed between said inlet and said outlet.

5. The fluid filtration system defined in claim 3, wherein said at least one layer of a porous medium is a bacterial retention medium.

6. The fluid filtration system defined in claim 3, wherein said at least one layer of a porous medium is a viral retention medium.

7. The fluid filtration system according to one of the preceding claims further including:
a) a filtration device, said filtration device having a filter media dividing said filtration device into an up-stream side and a downstream side,
b) a storage container for holding filtered fluid,
c) a second conduit connected between the downstream side of said filtration device and said storage container, and
d) a second vent line in fluid communication with the downstream side of said filtration device and selectively open to atmosphere.
